(19) Europäisches Patentamt
European Patent Office
Office européen des brevets

(11) Publication number: **0 442 231 A1**

(12) **EUROPEAN PATENT APPLICATION**

(21) Application number: **90403621.7**

(22) Date of filing: **17.12.90**

(51) Int. Cl.5: **G01N 33/543**, G01N 33/563, G01N 33/545

(30) Priority: **13.02.90 US 479253**

(43) Date of publication of application: **21.08.91 Bulletin 91/34**

(84) Designated Contracting States: **AT BE CH DE DK ES FR GB IT LI LU NL SE**

(71) Applicant: **EPITOPE, INC.**
**15425-E S.W. Koll Parkway**
**Beaverton Oregon 97006(US)**

(72) Inventor: **Thieme, Thomas R.**
**7020 Corvalis Road**
**Independence, Oregon 97351(US)**
Inventor: **Ferro, Adolph**
**5868 Suncreek Drive**
**Lake Oswego, Oregon 97035(US)**
Inventor: **Fellman, Jack**
**7615 S.W. View Pt. Terrace**
**Portland, Oregon 97219(US)**
Inventor: **Gavojdea, Stefan**
**18188-C N.W. Walker Road**
**Beaverton, Oregon 97006(US)**
Inventor: **Hinrichs, David**
**151 Del Prado**
**Lake Oswego,Oregon 97034(US)**

(74) Representative: **Clisci, Serge**
**S.A. FEDIT-LORIOT CONSEILS EN PROPRIETE INDUSTRIELLE 38, avenue Hoche**
**F-75008 Paris(FR)**

(54) **Stick probe device for detection of antibodies.**

(57) Disclosed is an article for use in an immunodiagnostic assay comprising an elongated stick having at least one portion with a non-liquid absorbing, non-porous surface, containing a ligand capable of binding with an immunoglobulin and a method for isolating an immunoglobulin from a body fluid comprising contacting the body fluid with a non-liquid-absorbing, non-porous substrate capable of binding with the immunoglobulin.

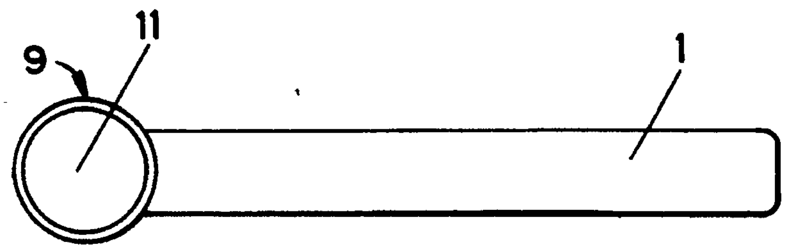


FIG. Ia

EP 0 442 231 A1

This invention relates to the detection, for screening and diagnostic purposes, of antibodies in body fluids such as saliva. In particular, this invention relates to the detection of antibodies which are present in low amounts in such fluids or where small amounts of such fluids are available and yet the antibodies present have antigenic specificities characteristics of particular disease states, and are of diagnostic value.

Body fluids of mammals, such as serum, plasma, saliva, tears, urine, milk, seminal fluid, synovial fluid, etc. can contain antibodies which are useful in the diagnosis of diseases, including those of bacterial and viral infection and of autoimmune origin. Body fluids, such as saliva, have significant advantage over serum and plasma as sources of these diagnostically valuable antibodies since they can be obtained without the facilities and hazards attendant with the taking of blood samples. However, often the concentration of antibodies is so low in these fluids as to make conventional tests for antibodies impractical.

Saliva, in particular, presents problems as a diagnostic indicator. These problems stem from the low concentration of antibodies in saliva and the inconvenience of collecting and processing quantities of saliva sufficient for a reliable diagnostic test, which can be quickly performed.

In one aspect, the present invention is directed to an article for use in an immunodiagnostic assay comprising an elongated stick having at least one portion with a non-liquid absorbing, non-porous surface containing a ligand capable of binding with an immunoglobulin.

In another aspect, the present invention is directed to a method for isolating a immunoglobulin from a body fluid comprising the sequential steps of contacting the body fluid with a non-liquid-absorbing, non-porous substrate capable of binding with the immunoglobulin and washing excess fluid from the substrate.

In another aspect, the present invention is directed to a method for isolating an antigen from a body fluid comprising the sequential steps of contacting the body fluid with a non-liquid-absorbing, non-porous substrate capable of binding with the antigen and washing excess fluid from the substrate.

The invention also contemplates a kit for detecting antibodies in saliva for diagnostic tests comprising an elongated stick having at least one portion with a non-liquid absorbing, non-porous surface containing a ligand capable of binding with an immunoglobulin; and reagents comprising buffer, biotinylated antigen solution, enzyme-linked avidin solution, an enzyme substrate capable of reacting with the enzyme to form a visibly detectable reaction product, and positive and negative control solutions.

A further aspect of the present invention is directed to a method for recovery of an immunoglobulin from a test fluid comprising the sequential steps of contacting the test fluid with a non-liquid-absorbing, non-porous substrate capable of binding with the immunoglobulin and washing excess fluid from the substrate; placing said substrate in an elution medium for the immunoglobulin; and, treating the eluate with a neutralizing buffer.

Fig. 1a is a top view and Fig. 1b is a side view of a preferred embodiment of the present invention. Fig. 2a is a side view and Fig. 2b is an end view of another preferred embodiment. Figs. 3, 4 and 5 are side views of various other preferred embodiments of the present invention.

A preferred embodiment of the present invention comprises an article for use in an immunodiagnostic assay comprising an elongated stick having at least one portion with a non-liquid absorbing, non-porous surface containing a ligand capable of binding with an immunoglobulin, wherein the portion is at one end of the stick. In another embodiment, the stick has the shape of a tongue depressor. In a further embodiment, the stick comprises an end portion that is bulbous. The stick is from about 50 to 150 mm in length, preferably about 76 mm in length. The stick is comprised of any substance that is convenient to manipulate by hand, preferably plastic. Materials such as wood, cardboard, or steel are other examples.

Another preferred embodiment of the present invention is directed to an article for use in an immunodiagnostic assay comprised of a stick having at least one end portion having affixed thereon a bead comprised of a substance having protein-binding properties, such that the bead and stick are two fixed elements. In another embodiment, the bead is affixed to said stick with glue. In a further embodiment, the bead is affixed to said stick by means of talons projecting from the end portion of said stick. The bead is from about 1.27 to 19.05 mm in diameter, preferably about 6.35 mm in diameter. The bead can be non-spherical. The bead is comprised of any substance suitable for the binding of antibody-binding substances, preferably polystyrene. Generally, the bead has a hard non-liquid-absorbing, non-porous, surface on which suitable chemical functional groups can be provided for covalent or non-covalent binding of proteins. This can include plastics which can be co-polymerized with monomers providing a carboxyl group such as methacrylic acid, acrylic acid or maleic anhydride. Amino groups can be provided with amino styrene monomers or monomers with primary amines on the aliphatic ethylene backbone. Another method is to coat an underivatized bead with a latex paint containing suitable chemical function groups for protein binding. In another embodiment, more than one bead is held by said talons. For example, one stick is equipped with three sets of talons, each set consisting, in turn, of three talons. Each set fixes one bead to the stick. Each bead is derivatized with a different protein, affording positive and negative controls as well as a test bead. In

another embodiment, the ligand is Protein A. In another embodiment, the ligand is selected from the group consisting of Types II, III, IV, V, and VI Fc receptor proteins.

Another preferred embodiment of the present invention is directed to a method for isolating an immunoglobulin from a body fluid comprising the sequential steps of contacting the body fluid with a non-liquid-absorbing, non-porous substrate capable of binding with the immunoglobulin and washing excess fluid from the substrate; contacting and incubating the substrate with a solution of an antigen and washing excess solution from the substrate; contacting and incubating the substrate with a compound capable of binding the antigen and washing excess solution from said substrate; and, contacting and incubating the substrate with a material capable of reacting with the compound to produce a visibly detectable reaction product. In another embodiment, the substrate capable of binding immunoglobulin is contacted with the body fluid by placing the substrate in a patient's mouth. The substrate can comprise protein A. The substrate can also comprise a ligand selected from the group consisting of Types II, III, IV, V, and VI Fc receptor proteins. In another embodiment, the antigen is biotinylated and the compound capable of binding the antigen is enzyme-linked avidin. In another embodiment, the antigen is HIV-1.

Another preferred embodiment of the present invention is directed to a method for isolating an antigen from a body fluid comprising the sequential steps of contacting the body fluid with a non-liquid-absorbing, non-porous substrate capable of binding with the antigen and washing excess fluid from the substrate; contacting and incubating the substrate with a solution of an antibody capable of binding said antigen and washing excess solution from said substrate; contacting and incubating the substrate with a solution of a compound capable of binding the antibody and washing excess solution from said substrate; and, contacting and incubating the substrate with a material capable of reacting with the compound to produce a visibly detectable reaction product. In another embodiment, the substrate capable of binding the antigen is contacted with the body fluid by placing the substrate in a patient's mouth. The substrate can comprise an anti-antigen protein bound by a ligand. In a further embodiment, the ligand is protein A or the substrate can comprise an anti-antigen protein bound by a ligand selected from the group consisting of Types II, III, IV, V, and VI Fc receptor proteins. In another embodiment, the antibody is a Fab fragment of a monoclonal antibody lacking the Fc portion which binds said substrate and capable of binding said antigen. The anti-antigen protein and the Fab antibody are specific for different epitopes of said antigen. In another embodiment, the antibody is biotinylated and the compound capable of binding the antibody is enzyme-linked avidin. In another embodiment, the antigen is HIV-1.

In another embodiment, the invention also contemplates a kit for detecting antibodies in saliva for diagnostic tests comprising an elongated stick having at least one portion with a non-liquid absorbing, non-porous surface, having a ligand capable of binding with an immunoglobulin; and, reagents comprising buffer, biotinylated antigen solution, enzyme-linked avidin solution, enzyme substrate capable of reacting with the enzyme to a visibly detectable reaction product, a polyclonal or monoclonal antibody specific for an antigen and biotinylated Fab monoclonal antibody, and positive and negative control solutions.

Another preferred embodiment of the present invention is directed to a method for recovery of an immunoglobulin from a test fluid comprising the sequential steps of contacting the test fluid with a non-liquid-absorbing, non-porous substrate capable of binding with the immunoglobulin and washing excess fluid from the substrate; placing said substrate in an elution medium for the immunoglobulin; and, treating the eluate with a neutralizing buffer, wherein said elution medium is 0.1 molar citric acid buffer of pH 3.0 and said buffer is 1 molar Tris.

Another preferred embodiment of the present invention comprises a method of rapidly isolating antibodies from a body fluid via a stick probe device, said device preferably having the substance protein A, a protein which rapidly binds to antibody molecules, chemically linked to a polystyrene bead affixed to a plastic handle. Using the handle, the bead is placed in the mouth of the individual to be tested and kept under the tongue or the buccal space for several minutes. The procedure is similar to the familiar technique of taking body temperature with an oral thermometer. The antibodies are thus rapidly removed from the fluid as it comes in contact with the bead and the antibodies are also concentrated on the bead. After several minutes in the mouth, the bead, which is held by the handle, is washed by briefly dipping in a solution of phosphate buffered saline or in a stream of tap water. Oral antibodies remain bound to the bead by virtue of the protein A. The bead is then subjected to biotinylated antigen and other enzyme-substrate reagents. The bead can be rapidly dipped in the succession of reagents to facilitate washing and equilibration with each reagent. The final chromogenic color develops as a dark blue precipitate on the bead. The protein A bead, therefore, is the surface on which the antibodies are retrieved and concentrated from the mouth as well as the surface on which the color development for a positive test develops. The protein A bead eliminates the need for obtaining oral rinses, saliva, or sputum as a source of diagnostic antibody with attendant containment, handling, and exposure risks and provides these antibodies on a

surface suitable for unusually rapid enzyme-linked immunodiagnostics.

It is preferred that the beads are polystyrene, hydrazide beads, alkyl amine beads, or Sanger Reagent beads. Such beads are well known and commercially available. For example, underivatized polystyrene beads are obtained from commercial suppliers such as Precision Plastic Ball Co., Chicago, IL. The polystyrene beads are then chloromethylated according to the procedure outlined in British Patent 816583 (July 15, 1959) and described in Chemical Abstracts 53:23079 i, the disclosures of which are incorporated herein by reference. Hydrazide beads are reacted with excess glutaraldehyde or other bifunctional aliphatic or aromatic aldehyde at neutral pH. The aldehyde derivatized beads are then reacted with protein A at acid pH in an amine-free buffer. Finally, the beads, with covalently linked protein A, are treated with a reducing agent such as sodium borohydride (1 mg $NaBH_4$ per 4 gram of beads). Control beads (non-reactive with antibody) are produced by substituting gelatin for protein A in the above process. Sanger reagent beads are polystyrene beads that provide the Sanger reagent (1-fluoro-2,4-dinitrobenzene) on the end of an aliphatic chain copolymerized into the polystyrene bead surface. The Sanger reagent allows covalent coupling of proteins via their amino groups to the bead as shown in the scheme below.

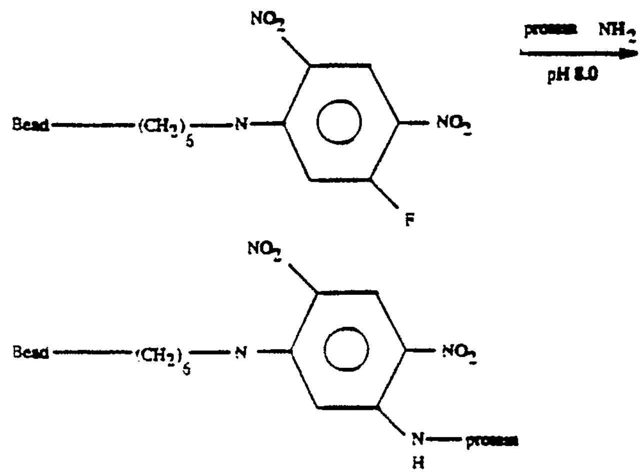


Alkylamine beads provide a primary amine covalently attached to the bead through copolymerization. This amine is used to link a protein to the bead in the following way: the alkylamine bead is reacted overnight with succinic anhydride in phosphate buffer of pH 6 (1 gm succinic anhydride per 2 grams of beads) as depicted below.

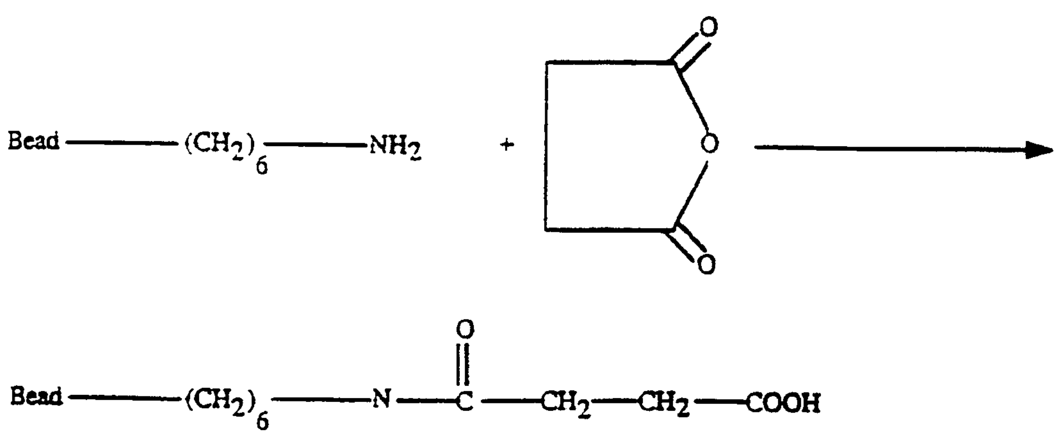

Carboxyl groups on the bead surface are then used to form an amide with amino groups available on the protein using a standard carbodiimide reaction. A typical carbodiimide would be ethyl-3-(3-dimethylaminopropyl)-carbodiimide. The carboxyl-group-derivatized beads (10 beads) are incubated for 2 hours in 5 ml of the carbodiimide (200 mg) at pH 10. The beads are immediately washed in distilled water and incubated with the protein solution (5 mg/5ml) for 1 hour. All reactions are at room temperature. All of

the derivatized beads cited above contain approximately 3 micromoles of the functional group on the surface of a 6.35 mm specular non-porous bead according to manufacturers specifications.

Other types of beads are suitable for the purposes of this invention if they have low non-specific binding of proteins and functional groups such as hydroxyl, carboxyl, or amino groups or unique chemistries which allow the covalent binding of protein A or other proteins which bind antibodies. Polystyrene beads which contain on their surface chemically reactive groups for covalent bonding of protein are available from Pierce Chemical Company, Rockford, Illinois. Such beads include those containing reactive hydrazide groups, alkylamine groups, or Sanger Reagent. Shapes other than spherical for the polystyrene probe are also contemplated such as a paddle shape, and may have advantages in the washing and immunoassay development steps. If a flat surface is present on such a probe, the protein A can be applied to the surface in the form of a " + " (plus or positive) sign as shown in Fig. 3. If the polystyrene forming the paddle 5 in Fig. 3 is derivatized and activated (e.g. by a carbodiimide), a solution of protein A can be used to paint a " + " sign on the paddle. After reaction with the protein A the remainder of the paddle is reacted with gelatin or any other protein which does not specifically react with antibody such as serum albumin. After reaction with the patient sample, biotinylated disease-specific antigen, enzyme-linked streptavidin (or enzyme-linked antigen) and appropriate chromagen, a positive patient sample will produce a " + " sign on the paddle while a negative sample will produce no such sign despite the possible presence of a high background due to interfering substances in the mouth. Color in the form of a " + " indicates antibody derived reactivity. If a bead is used, the preferable size is 6.35 mm in diameter. Beads can range in size from 1.27 to 19.05 mm in diameter although 2.54 to 12.7 mm is more convenient and economical. The preferable size for the paddle is 12.7 mm, although 6.35 to 19.05 mm will function satisfactorily. An additional form for the probe contemplated here is a plastic stick having a spoon-like or well-like end portion. The handle is about 76 mm long and 10 mm in width and 1mm in thickness although dimensions may vary. The spoon-like end has an inside diameter of 15 mm and an outside diameter of about 17 mm. The volume of the well is about 0.32 ml. At the bottom of the well is a 100 micron thick microporous membrane preferably of polyvinylidene difloride with a 3 mm circle of Protein A or other antibody-binding protein covalently bound to the membrane. Binding of the Protein A is carried out by placement of 2 $\mu$l of a 1 mg/ml solution of Protein A in a non-amino buffer such as phosphate buffer on the membrane. Other membranes are reacted with gelatin (providing a negative control) or biotinylated gelatin providing a positive reagent control.

The amount of protein A linked to the bead, paddle, or membrane is sufficient to bind an optimal amount of the antibodies in the body fluid such that a reaction product detectable by eye is produced. These probes provide an adequate surface size for the subsequent colorimetric assay.

Protein A is a protein isolated from the cell wall of the bacteria Staphylococcus aureus (Cowan strain 1). Protein A (also called Type 1 Fc receptor) is available commercially as a protein isolated from this bacteria and also in a recombinant form expressed in other bacteria. The recombinant protein as expressed in E. coli is obtained in purified form from Repligen Corporation, Cambridge, Massachusetts. Protein A can be covalently linked to the bead by any of several commonly used chemical reactions. Some examples of these techniques are the following: Amino groups or carboxyl groups are introduced onto the bead by co-polymerization with monomers containing these functional groups such as methacrylic acid or aminostyrenes. These derivatized beads are then coupled to carboxyl groups or amino groups on the protein by the use of the commonly used carbodiimide chemistries or the use of such commonly used leaving groups (the conjugate base of a strong acid) as N-hydroxysuccinimide which is introduced onto the amino derivatized bead with O-bromoacetyl-N-hydroxysuccinimide. Other known techniques can be used to covalently bind protein A to polystyrene beads or beads of other compositions.

Other proteins which rapidly and avidly bind antibodies can be used in accordance with the present invention instead of protein A. These proteins include certain proteins isolated from group A streptococci (Type II Fc receptor), protein G (Type III Fc receptor) isolated from most human C and G streptococcus strains, Type IV Fc receptor isolated from some strain G streptococcus strains, and Types V, VI Fc receptors isolated from Streptococcus zooepidimicus. Each of these proteins has certain advantages over the others in its strength of binding to different subclasses of immunoglobulins and immunoglobulins from different mammalian species.

A plastic, i.e., polystyrene, handle is attached to the bead with a small drop of glue. If the device is to be inserted in the mouth, a non-toxic glue is required. A methylmethacrylate glue such as used in fabrication of dental devices is preferable. Wood or metal handles are also usable. The handle is preferably about 76 mm long. Handles between 50 and 150 mm are also convenient. As shown in Figs. 2 and 3, an alternative handle is a plastic stick 1 with nylon or polystyrene talons or prongs 3 which hold the bead by a pinching action. As shown in Fig. 4, the device can also be formed as one unit such as one similar in shape

to a tongue depressor having stick 1 and paddle-shaped end 5, or as shown in Fig. 5, a stick 1 with wells 7 containing membrane bound protein A. Using several stick-bead probes, or one stick with more than one bead attached (e.g. by increasing the number of talons on the stick as shown in Fig. 3), several beads for multiple assays can be recovered at the same time from one subject or an additional bead without protein A (rather gelatin) can serve as a negative control. In yet another embodiment as shown in Figs. 1a and 1b, stick 1 has spoon-shaped end 9, in which membrane 11 is attached. The membrane is a microporous membrane such as polyvinylidene difloride to which is bound protein A.

The stick probe described here fulfills the essential requirement of a device which will (1) contain adequate surface area for binding, (2) allow easy observation of the bead, (3) allow the body fluid to be tested with adequate contact surface between solutes in the body fluid and the protein A attached to the beads, (4) allow the sequential reaction with and washing off of the reagents used to perform the colorimetric tests of the antibodies in the body fluid.

After binding of the antibodies to the bead as indicated above, excess amounts of the body fluid are removed from the bead by washing with a suitable aqueous buffer. The bead with bound antibody is then used as a substrate to assay for the antigenic specificity of the bound antibody.

Several methods are available for the assay of the antigenic specificity of isolated antibodies. These include the use of enzymes such as horseradish peroxidase or alkaline phosphatase covalently linked to the antigens of interest. This technique is illustrated in T. Kitagawa et al., "Enzyme coupled immunoassay of insulin using a novel coupling reagent," Journal of Biochemistry, 79:233236, 1976. These enzymes are used to develop color reactions which indicate the presence of the antigen. Another method which is available is the covalent binding of biotin to the antigen as in G. R. Dreesman et al., U.S. Patent 4,535,057; D. A. Fuccillo, "Application of the avidin-biotin technique in microbiology," Bio-Techniques, 3:494-501, 1985; and M. Wilchek et al., "The use of the avidin-biotin complex as a tool in molecular biology," Methods of Biochemical Analysis, 26:1-45. It is bound with very high avidity (Kd 10-15M) by the protein avidin. Four biotin molecules are bound per avidin molecule. When enzymes such as horseradish peroxidase or alkaline phosphatase are chemically linked to the avidin molecule, the avidin-biotin interaction can constitute a molecular bridge between the antigen of interest and the enzyme which is used to develop the color reaction that indicates the presence of the antigen. Because each avidin molecule has four biotin binding sites, the number of enzyme molecules per antibody molecule is increased in the presence of excess biotinylated antigen and the sensitivity of the assay is increased.

The chemical bond between the antigen and biotin molecule can be formed by a number of different chemical reaction sequences which are available. These reactions typically utilize N-hydroxysuccinimidyl or iodo leaving groups to facilitate binding to derivative amino or sulfhydryl groups in the protein structures of the antigen. Typical reagents are shown below:

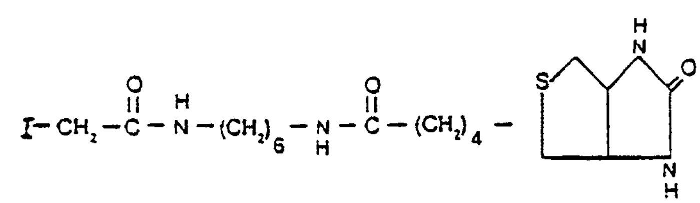

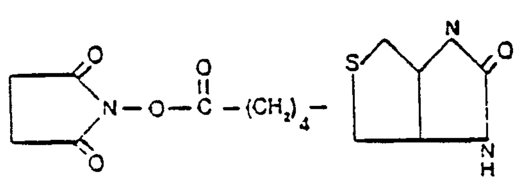

Carbohydrate antigens can be biotinylated by the reaction of biotin hydrazide with aldehyde groups produced in polysaccharide structures after reaction with periodate. The selection of the best biotinylation system among these and other available chemical reactions depends on the particular nature of the antigen being tested. HIV antigen can be biotinylated according to the following scheme:

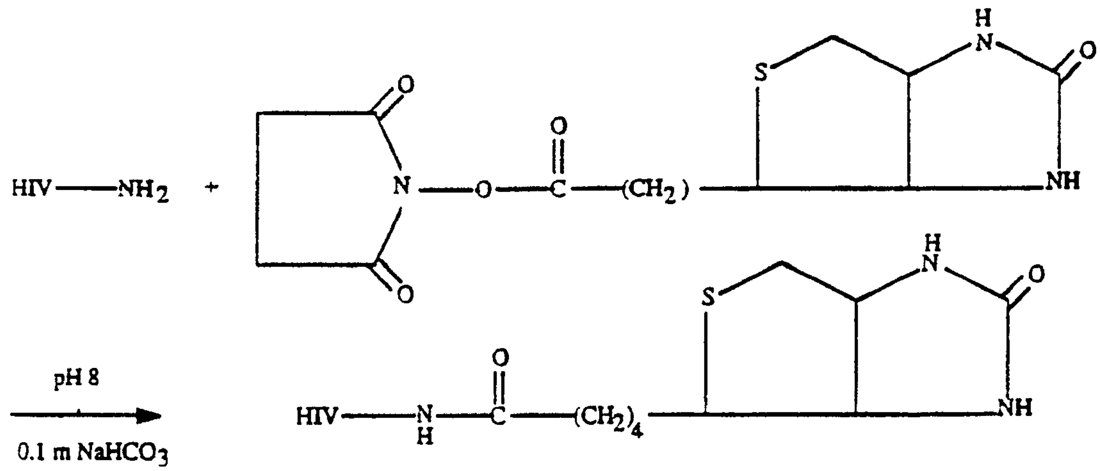


The present invention represents a unique application of the use of the biotin-avidin reaction to link antigen with an enzyme detection system. The antigen has been immobilized as it comes in contact with the bead upon which specific antibody has in turn been immobilized by protein A.

In the presence of excess biotinylated antigen, many avidin-enzyme conjugates can bind to the bead for every antibody immobilized by protein A. This gives the technique its high sensitivity.

The enzyme coupled to the avidin determines the colorimetric assay used in the detection of antigen, which ultimately is a test for the presence of certain types of antibody in the biological fluid. Two of the more commonly used enzymes are alkaline phosphatase and horseradish peroxidase. These enzymes can be chemically bonded and linked to the avidin molecule utilizing commonly used chemical pathways for protein-protein covalent linkage including the use of glutaraldehyde or the use of other homobifunctional (containing identical binding groups) and heterobifunctional (containing dissimilar binding groups) reagents such as disuccinimidyl suberate and succinimidyl-N-(4-carboxy-cyclohexylmethyl)-maleimide (S. Yoshitake et al., "Conjugation of glucose oxidase from Aspergillus niger and rabbit antibodies using N-hydroxy-succinimide ester of N-(4-carboxy-cyclohexylmethyl)-maleimide," Eur. J. Biochem, 101:395-399). The binding groups on these agents are chemically reactive functional groups which can form covalent bonds with reactive groups on proteins such as amino or sulfhydryl groups. The chemistries cited above utilize the reactivity of the amino group of proteins with the succinimidyl ester, and the reactivity of the free SH group of proteins with the maleimide moiety.

If horseradish peroxidase is used as the detection enzyme, typical substrates would be diaminobenzidine, 4-chloro-1-naphthol, or 3,3',5,5'-tetramethylbenzidine (Sheldon, E. L., et al., "Use of nonisotopic M13 probes for genetic analysis: application to HLA class loci," Proc. Natl. Acad. Sci., 83:9085-9089). When any of these materials is mixed with hydrogen peroxide and exposed to the enzyme, a darkly colored polymeric material is formed. This colored product is fast, i.e., insoluble and precipitating on the solid support. The production of this colored precipitate indicates the presence of the enzyme and in the context of this invention the presence of antibodies specific for the antigen being investigated. Other materials producing highly colored precipitates are used to indicate the presence of alkaline phosphatase such as a mixture of 5-bromo-4-chloro-3-indolyl-phosphate and nitroblue tetrazolium.

In the present invention, the capture of antibodies by the Protein A attached to the bead occurs in minutes with a total assay time of about 10 minutes. The use of the stick probe technique also lends speed and convenience to the present invention, and permits the concentration of antibodies from a large volume of fluid.

The assay time can be additionally shortened by eliminating the biotin-avidin component of the reaction and using a preparation of HIV-I antigens directly covalently linked to horseradish peroxidase. Such protein-protein linkages can be produced by standard procedures such as glutaraldehyde coupling or the use of heterobifunctional cross-linking reagents such as the N-hydroxysuccinimide ester of 4-(N-maleimidomethyl) cyclohexane-1-carboxylic acid as previously described. The use of direct HIV-1-peroxidase conjugates shortens the reaction time, while the sensitivity of the test is somewhat less.

This technique is readily adaptable to the detection of any oral antibodies to infectious agents or autoantibodies, whenever such antibodies occur in oral secretions. Examples include oral antibodies to HTLV-I, Hepatitis A, or Hepatitis B detected by the use of the appropriate biotinylated or peroxidase conjugated antigen. A mixture of any of these antigens can be used to screen for persons infected with one

or more of these agents by this simple oral test. For example, antigens from the viruses HTLV-1, HIV-1 and HIV-2 can be mixed together on an equal weight basis and biotinylated as described herein. If such a mixture is used as the antigen source for reaction with a protein A-derivatized bead after exposure to saliva in a patient's mouth, a positive color development after treatment with avidin-peroxidase would indicate that the patient possessed antibodies to one or more of the three viruses. Subsequent testing for antibodies to the individual viruses would be indicated. Such a test would obviously have value in testing large populations where the frequency of antibodies to any of these viruses is low.

To more clearly describe the present invention, the following non-limiting examples are provided. In the examples, all parts and percentages are by weight unless indicated otherwise.

**EXAMPLE I**

Preparation of alkylamine, carboxyl, carboxyhydrazide and hydroxy derivatized polystyrene beads is performed in this example.

The procedure is as follows: 100 grams of 1/4" beads in 84 grams of methylal are treated successively with 200 cc ethylene chloride, 33 grams of paraformaldehyde, and 40 grams of $AlCl_3$. The reaction is stirred at 55° C for 2 hours. After cooling to room temperature, the beads are washed with ethylene chloride and tetrahydrofuran (THF). In the final product, approximately 21% of the benzene rings are chloromethylated at the para position.

The chloromethylated polystyrene beads are then converted to the alkylamine derivatized beads by reaction with 50 grams of 1,6 hexane diamine in 200 cc of ethylene chloride for 2 hours at room temperature followed by washing in ethylene chloride and THF.

Carboxylated beads are obtained by reacting the chloromethylated polystyrene with 50 gm of 6-amino caproic acid in ethylene chloride followed by washing.

The above carboxylated polystyrene beads are further modified to form the corresponding hydrazide by first treating the carboxylated polystyrene with 50 cc of thionylchloride under reflux for 3 hours to form the acid chloride. Following this, the beads are separated and washed with ethylene chloride and then treated with 25 cc hydrazine in THF at room temperature for 2 hours. The beads are then separated and washed with THF.

Hydroxylated polystyrene beads are obtained by boiling the chloromethylated beads in water for 3 hours.

Alternative methods for derivatizing polystyrene particles or microspheres are disclosed in U.S. Patents 4,480,042 (10/30/84) and 4,140,662 (2/20/79).

With the above derivatized beads, subsequent covalent binding of protein A can be accomplished as outlined previously.

**EXAMPLE II**

Preparation of the stick probe device is performed in this example. For protein A coupling, 25 beads containing approximately 3 micromoles of hydrazide function per bead as prepared in Example I are incubated in 5 milliliters of 12.5 percent glutaraldehyde solution in 0.1 molar phosphate buffer pH 7.0 with gentle shaking for 2 hours. Beads are then washed successively with 100 milliliters of distilled water and 20 milliliters of 0.1 molar phosphate buffer using a Buchner funnel. Two and one-half milligrams of protein A are dissolved in 5 milliliters of 0.1 molar phosphate buffer pH 6.0 and incubated with the glutaraldehyde-reacted beads for 20 hours at room temperature. One milligram of cyanoborohydride is added to the mixture immediately after combination of the beads and protein A and prior to incubation. After the 20 hours of reaction, the beads are washed with 100 milliliters of 0.1 molar phosphate buffer and 50 milliliters of 0.1 molar sodium bicarbonate pH 8.0. The beads are then suspended in 5 milliliters of 0.1 molar sodium bicarbonate containing 1 mg of sodium borohydride. The mixture is incubated for 15 minutes with gentle shaking. The beads are then washed with 100 milliliters of sodium carbonate followed by 100 milliliters of distilled water. The beads are then "blocked" to prevent non-specific absorption of proteins by incubating them for 24 hours in a blocking buffer consisting of 2.5% gelatin, 0.5% BSA (bovine serum albumin), 0.3% Tween 20 detergent (available from chemical suppliers such as Sigma Chemical) and 1 molar TRIS i.e. Tris (hydroxy methyl amino methane), pH 8.0. Beads can be stored in blocking buffer or blotted dry and kept in closed tubes.

**EXAMPLE III**

In this example, assay of sample fluid is performed to test for HIV-I antibodies in the oral cavity of a patient. One bead, as prepared in Example II, is placed in the mouth of a known seropositive subject. The bead is kept in the mouth by the subject for 2 minutes, occasionally moving it from under the tongue to the buccal space. The bead is removed from the mouth and agitated in 10 ml of phosphate buffered saline (PBS) in a small container. The bead is then submerged in 2 ml of a solution containing 5 micrograms of biotinylated HIV-I antigen in a buffer of 1% gelatin, 0.3% Tween 20, and 1 molar TRIS, pH 8.0, i.e., a rapid assay diluent (RAD) buffer. The bead is kept in this solution for up to 5 minutes and then rapidly washed 3 times in RAD buffer and once in PBS. The bead is then incubated for 5 minutes in 2 ml of RAD buffer containing 5 mg of enzyme-labelled avidin prepared as previously described such as Strepavidin-horseradish peroxidase conjugate (available from suppliers such as Kirkegaard and Perry Labs, Gaithersburg, Maryland). The bead is again washed in RAD buffer and PBS, and incubated with a chromogenic substrate for peroxidase, i.e., 3,3' ,5,5'-tetramethylbenzidine with 0.03% $H_2O_2$ and a precipitation enhancer (dioctyl sulfosuccinate). The color reaction develops immediately, giving a dark blue colored bead while control beads used by HIV-I seronegative subjects remain an opaque white color. The wash steps in RAD buffer and PBS can be eliminated by simply holding the bead in a gentle stream of tap water. Total assay time is about 10 minutes.

Biotinylation of HIV-1 antigen used in the foregoing process is accomplished as follows: HIV-1 lysate; a detergent (Nonidet-P40) extract of virus produced on a human cell line available from Genetic Systems Corp., Seattle, WA. is passed over a 0.5 x 10 cm gel filtration column (Bio-Gel P-6 is obtained from Bio-Rad Corp., Richmond, CA) to remove the TRIS buffer contained in the extract and replace it with buffer for coupling of biotin (0.1 M sodium bicarbonate, 0.5 M sodium chloride). 200 micrograms of viral lysate (100 microliters) are passed through the column and recovered in 300 microliters of coupling buffer. Approximately 0.5 mg of N-hydroxysuccinimide biotin is added to the solution with overnight incubation. Biotin compounds not coupled to the viral proteins are removed by passing the product over another 0.5 x 10 cm gel filtration column equilibrated with phosphate buffered saline.

A more definitive and sensitive interpretation of the results can be obtained if two beads are placed in the mouth of the subject to be tested. One bead is derivatized with protein A as in Example II and the second with gelatin or another protein which does not react with antibodies (e.g. BSA). Both beads are exposed to antigen and the other reagents described above. The protein A bead is distinguished from the gelatin bead by a mark of colored paint on the stick holding the bead. After reaction with chromogen, a positive reaction is indicated by a darker color development on the protein A bead than the gelatin bead.

Beads possessing alkylamine functional groups as described in Example I are derivatized with protein as follows: 15 beads are placed in 25 ml of 0.05 M sodium phosphate buffer pH6. One gram of succinic anhydride is added and the mixture gently shaken overnight. The 15 beads are then washed in 100 ml of water. A 5 ml aqueous solution of 1-ethyl-3-(3-dimethylaminopropyl)carbodiimide at pH10 is prepared and immediately added to the beads with incubation for 2 hours. Beads are then washed in 100 ml water and immediately added to a solution of the protein to be coupled (either protein A or gelatin) at a concentration of 1 mg per ml in 0.1 M sodium phosphate buffer pH7. Beads are again washed in water and blocked as in Example II.

**EXAMPLE IV**

In addition to its use as a surface for direct immunodiagnostic assays, the protein A bead recovered form the mouth of the subject can be used to recover soluble antibodies for testing. A stick probe prepared as in Example I is placed in the mouth of a subject as in Example II. After removal from the subject's mouth, the protein A bead is placed in a 0.1 molar citric acid buffer pH 3.0 (or other suitable elution medium) for 30 minutes. Under these conditions any antibodies will be released from the bead. After removing the bead, the citric acid eluate is neutralized to pH 7.0 with a suitable base such as 1 molar TRIS. The resulting antibody solution can be used in any immunodiagnostic assay performed as a primary test or confirmation of an earlier assay for corroboration.

**EXAMPLE V**

This example demonstrates direct antigen detection in accordance with the present invention. An antibody specific for the antigen of interest is immobilized by the protein A bound bead of Example I. In this example, a mouse monoclonal antibody against the p24 core antigen of HIV-1 is reacted with a protein A bead by incubating the bead (held by its handle) with a 0.1 mg/ml solution of the antibody in 0.1 M sodium bicarbonate solution (pH 8) for 10 minutes at room temperature. The bead complex is then exposed to a

test fluid to react with any antigen present. This is accomplished by inserting the bead (coated with protein and monoclonal antibody) into the subject's mouth for 2-5 minutes or into 5 milliliters of a freshly obtained mouth rinse from the subject for the same time. The rinse is obtained with a 1-3% saline solution of neutral pH containing .10% benzoic acid or sorbic acid as an antibacterial agent. Next, the bead is incubated with a second antibody that is prepared as follows. A mouse monoclonal antibody (distinct from the one above but reacting with the same protein, p24) is modified so that it is a biotinylated Fab fragment (lacking the Fc part of the molecule which binds to Protein A, but retaining its ability to bind antigen) and it is then used to bind to the HIV-1 antigen/antibody/protein-bound bead complex. In a typical preparation of this antibody, 5 mg of a mouse IgG antibody specific for HIV-1 p24 is dissolved in one milliliter of a 0.1 M sodium phosphate buffer (PBS) containing $4x10^{-3}$M ethylene diaminetetraacetic acid (pH 7.5). 2-Mercaptoethanol is then added to a final concentration of 0.01 M. The proteolytic enzyme papain is then added to a concentration of 100 micrograms per ml. The mixture is incubated at 37°C overnight. The reaction is terminated by making the solution 0.01M in iodacetamide. Fab monoclonal antibody is separated from intact antibody and free Fc portions of antibody by passing the mixture over a 1 x 5 cm protein A agarose column (Bio-Rad Corp.). This Fab fragment of the mouse monoclonal antibody is then biotinylated as described in Example II. This biotinylated Fab mouse monoclonal antibody against HIV-1 p24 is then incubated in PBS at 0.1 mg/ml with the bead described above containing the HIV-1 antigen/antibody/protein-bound complex on its surface for 2-5 minutes. The complex is then reacted with an avidin-enzyme detection reagent as in Example II. After washing, chromogenic detection is carried out as in Example II. The monoclonal antibody Fab fragment will only bind to the bead complex and thus color development will only be seen, if the antigen is present.

## Claims

1. An article for use in an immunodiagnostic assay comprising an elongated stick having at least one portion with a non-liquid absorbing, non-porous surface containing a ligand capable of binding with an immunoglobulin.

2. The article of claim 1, wherein the portion is at one end of the stick.

3. The article of claim 2, wherein said portion is bulbous.

4. The article of claim 2, wherein said stick is from about 50 to 150 mm in length.

5. The article of claim 2, wherein the portion comprises a bead having a diameter of about 1.27 to 19.05 mm.

6. The article of claim 5, wherein the bead and stick are two fixed elements.

7. The article of claim 6, wherein the bead is affixed to said stick by means of talons projecting from the stick.

8. The article of claim 5, wherein the bead is comprised of polystyrene.

9. The article of claim 7, having more than one bead.

10. The article of claim 1, wherein the ligand is Protein A.

11. The article of claim 1, wherein the ligand is selected from the group consisting of Types II, III, IV, V, and VI Fc receptor proteins.

12. A method for isolating a immunoglobulin from a body fluid comprising the sequential steps of contacting the body fluid with a non-liquid-absorbing, non-porous substrate capable of binding with the immunoglobulin and washing excess fluid from the substrate.

13. The method of claim 12, further comprising the steps of:
contacting and incubating the substrate with a solution of a antigen and washing excess solution from the substrate;
contacting and incubating the substrate with a compound capable of binding the antigen and

washing excess solution from said substrate; and
contacting and incubating the substrate with a material capable of reacting with the compound to produce a visibly detectable reaction product.

**14.** The method of claim 13, wherein the antigen is biotinylated and the compound capable of binding the antigen is enzyme-linked avidin.

**15.** The method of claim 13, wherein the antigen is HIV-1.

**16.** The method of claim 13, wherein the substrate capable of binding immunoglobulin is contacted with the body fluid by placing the substrate in a patient's mouth.

**17.** The method of claim 13, wherein the substrate comprises protein A.

**18.** The method of claim 13, wherein the substrate comprises a ligand selected from the group consisting of Types II, III, IV, V, and VI Fc receptor proteins.

**19.** A method for isolating an antigen from a body fluid comprising the sequential steps of contacting the body fluid with a non-liquid-absorbing, non-porous substrate capable of binding with the antigen and washing excess fluid from the substrate.

**20.** The method of claim 19, further comprising the steps of:
contacting and incubating the substrate with a solution of an antibody capable of binding said antigen and washing excess solution from said substrate;
contacting and incubating the substrate with a solution of a compound capable of binding the antibody and washing excess solution from said substrate; and,
contacting and incubating the substrate with a material capable of reacting with the compound to produce a visibly detectable reaction product.

**21.** The method of claim 20, wherein the substrate capable of binding the antigen is contacted with the body fluid by placing the substrate in a patient's mouth.

**22.** The method of claim 20, wherein the substrate comprises an anti-antigen protein bound by a ligand.

**23.** The method of claim 22, wherein the ligand is protein A.

**24.** The method of claim 22, wherein the substrate comprises an anti-antigen protein bound by a ligand selected from the group consisting of Types II, III, IV, V, and VI Fc receptor proteins.

**25.** The method of claim 20, wherein said antibody is a Fab fragment of a monoclonal antibody lacking the Fc portion.

**26.** The method of claim 24, wherein the anti-antigen protein and the Fab antibody are specific for different epitopes of said antigen.

**27.** The method of claim 20, wherein the antibody is biotinylated and the compound capable of binding the antibody is enzyme-linked avidin.

**28.** The method of claim 20, wherein the antigen is HIV-1.

**29.** A kit for detecting antibodies in saliva for diagnostic tests comprising:
an elongated stick having at least one portion with a non-liquid absorbing, non-porous surface, having a ligand capable of binding with an immunoglobulin; and,
reagents comprising buffer, biotinylated antigen solution, enzyme-linked avidin solution, enzyme substrate capable of reacting with the enzyme to a visibly detectable reaction product, and positive and negative control solutions.

**30.** A kit as defined in claim 29, further comprising a polyclonal or monoclonal antibody specific for an

antigen and biotinylated Fab monoclonal antibody.

**31.** A method for recovery of a immunoglobulin from a test fluid comprising the sequential steps of:
contacting the test fluid with a non-liquid-absorbing, non-porous substrate capable of binding with the immunoglobulin and washing excess fluid from the substrate;
placing said substrate in an elution medium for the immunoglobulin; and,
treating the eluate with a neutralizing buffer.

**32.** The method of claim 31, wherein said elution medium is 0.1 molar citric acid buffer of pH 3.0.

**33.** The method of claim 31, wherein said buffer is 1 molar Tris.

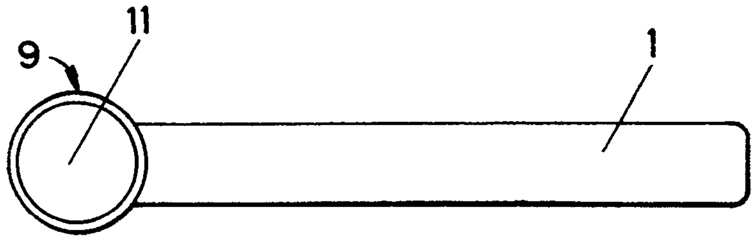


FIG. 1a

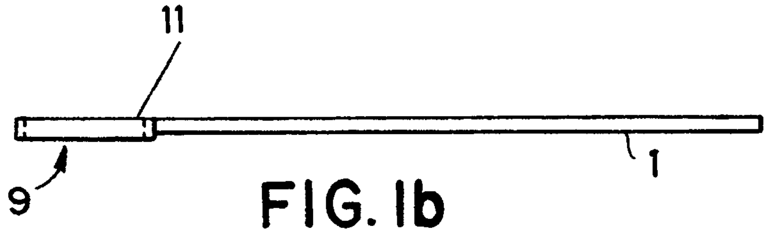


FIG. 1b

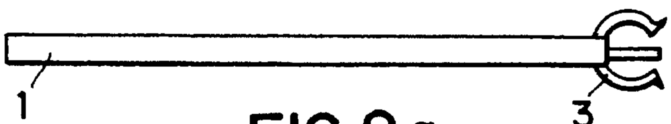


FIG. 2a

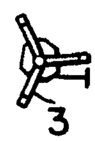


FIG. 2b

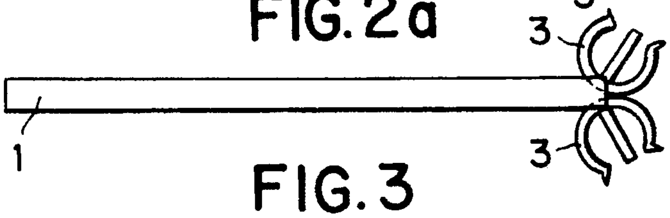


FIG. 3

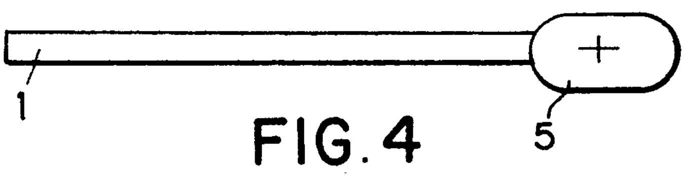


FIG. 4

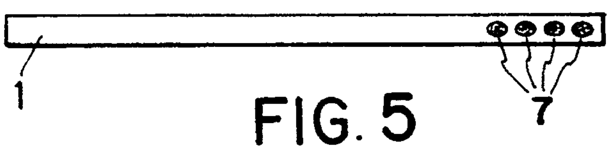


FIG. 5

European Patent Office

# EUROPEAN SEARCH REPORT

Application Number

**EP 90 40 3621**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | ZENTRALBLATT FÜR VETERINÄRMEDIZIN - REIHE B, vol. 29, no. 8, September 1982, pages 583-590, Berlin, DE; C. ALTANER et al.: "A simple and inexpensive method for detection of BLV infected cattle based on a modified ELISA principle" * Page 583 - page 584, line 23; figure 1 * | 1-3,10-15 | G 01 N 33/543<br>G 01 N 33/563<br>G 01 N 33/545 |
| Y | EP-A-0 346 119 (CAMBRIDGE BIOSCIENCE CO.) * Claims 1,5,7; example 1 * | 1-3,10-15 | |
| A | BIOTECHNOLOGY, vol. 5, July 1987, pages 697-703; M.D.P. BOYLE et al.: "Bacterial Fc receptors" * Whole document * | 1-3,10-15 | |
| X | US-A-4 135 884 (S.T. SHEN) * Whole document * | 19-20 | |
| A | EP-A-0 376 135 (W.R. GRACE & CO.) * Abstract; example 1; figure 1 * | 1-5 | |

TECHNICAL FIELDS SEARCHED (Int. Cl.5)

G 01 N

The present search report has been drawn up for all claims

| Place of search | Date of completion of search | Examiner |
|---|---|---|
| The Hague | 11 March 91 | MERLU B.H.A. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document